# EUROPEAN PATENT SPECIFICATION

(11) **EP 3 453 351 B1**
(45) Date of publication and mention of the grant of the patent: **08.09.2021**
(21) Application number: 16900959.4
(22) Date of filing: 26.07.2016
(51) Int. Cl.: A61B 17/86, A61L 31/02, A61L 31/14

(54) **POROUS TANTALUM METAL SCREW FOR FIXING BROKEN BONE, AND APPLICATION THEREOF**
PORÖSE TANTALMETALLSCHRAUBE ZUR BEFESTIGUNG VON GEBROCHENEN KNOCHEN UND ANWENDUNG DAVON
VIS MÉTALLIQUE POREUSE EN TANTALE POUR FIXER DES OS CASSÉS ET SON APPLICATION

(30) Priority: 06.05.2016 CN 201610297394; 06.05.2016 CN 201620406230 U
(43) Date of publication of application: 13.03.2019
(73) Proprietor: Zhao, Dewei, Dalian, Liaoning 116001 (CN); Wang, Benjie, Dalian, Liaoning 116001 (CN); Xie, Hui, Dalian, Liaoning 116001 (CN); Liu, Baoyi, Dalian, Liaoning 116001 (CN); Ma, Zhijie, Dalian, Liaoning 116001 (CN); Wang, Wei, Dalian, Liaoning 116001 (CN)
(72) Inventor: Zhao, Dewei, Dalian, Liaoning 116001 (CN); Wang, Benjie, Dalian, Liaoning 116001 (CN); Xie, Hui, Dalian, Liaoning 116001 (CN); Liu, Baoyi, Dalian, Liaoning 116001 (CN); Ma, Zhijie, Dalian, Liaoning 116001 (CN); Wang, Wei, Dalian, Liaoning 116001 (CN)
(74) Representative: Proi World Intellectual Property GmbH
(86) International application number: PCT/CN2016/091733
(87) International publication number: WO 2017/190437

(56) References cited:
- EP-A1- 2 700 371
- CN-A- 103 491 890
- CN-A- 103 519 880
- CN-A- 105 147 383
- CN-U- 201 743 761
- CN-U- 204 364 109
- US-A- 5 171 279
- US-A- 5 282 861
- US-A1- 2014 148 853
- DATABASE WPI Week 201626 Thomson Scientific, London, GB; AN 2016-18744C XP002795671, & CN 205 083 596 U (HUANG S) 16 March 2016 (2016-03-16)
- DATABASE WPI Week 201537 Thomson Scientific, London, GB; AN 2015-330441 XP002795672, & CN 104 352 276 A (PUTIAN COLLEGE AFFILIATED HOSPITAL) 18 February 2015 (2015-02-18)

## Description

### TECHNICAL FIELD

The present invention relates to a medical permanent implant, and in particular to a porous tantalum metal screw of medical implants used for treatment of femoral neck fracture.

### BACKGRONDART

Femoral neck fracture is a common clinical disease, especially for elderly patients with femoral neck fracture, they may have to face the fate of long-term bedridden or artificial joint replacement. Therefore, there is controversy about its treatment, most scholars believe that the treatment of fresh femoral neck fracture should follow the principle of early treatment, anatomical reduction and firm fixation; however, currently the internal fixation methods basically adopt the fixation with compressed hollow screws. At present, hollow screws made of titanium are widely used in clinical practice. Although certain clinical effects have been achieved at present, there are clinical defects such as no induction of bone formation, secondary removal, etc.

Porous medical metal implant materials have important and special purposes of treating bone tissue trauma and inducing bone tissue regeneration, these materials usually are porous stainless steel, porous titanium and so on. As a porous implant material used for the treatment of bone tissue trauma and defect, a porosity of the material should reach 30-85%, and pores are preferably all communicated and uniformly distributed, or the pores are partially communicated and uniformly distributed according to the need, so as to be consistent with the bone tissue growth of the human body, as well as to reduce the weight of the material itself to fit the human body implantation.

Metal tantalum has no toxic side effects on human body, and has good biocompatibility and mechanical properties. The porous material of the metal tantalum is expected to replace the traditional medical metal biomaterials and become the main biomaterials in the field of the treatment of femoral neck fracture. With the rapid development of medical science at home and abroad and the further understanding of tantalum as a human body implant material, the demands of people on the porous metal tantalum material of the human body implantation become more and more urgent, and the requirements on the porous metal tantalum material are also increasing. As porous medical implant metal tantalum, if it can have a high uniformly distributed and communicated pores, and physical and mechanical properties to adapt to the human body, it is an important material of connecting piece to ensure the normal growth of new bone tissue.

For the materials porous tantalum, US5282861 discloses an open cell tantalum material for cancellous bone implants and cell and tissue receptors and preparation thereof. This porous tantalum is made of commercial pure tantalum, and a carbon skeleton obtained by thermal degradation of polyurethane precursor is served as support. The carbon skeleton is a multi-dodecahedron in which a grid-like structure exists. The carbon skeleton is entirely distributed with micropores, and has a porosity as high as 98%, and then the commercial pure tantalum is bonded to the carbon skeleton by a chemical vapor deposition and penetrative method to form a porous metal microstructure, which is called a chemical deposition method for short. A thickness of a tantalum layer on the surface of the porous tantalum material obtained by this method is 40-60µm; and the specific gravity of the tantalum is about 99% and that of the carbon skeleton is about 1% in the entire porous material. It is further documented that the porous material has a compressive strength of 50-70MPa, an elastic modulus of 2.5-3.5GPa, a tensile strength of 63MPa, and a plastic deformation of 15%.

At present, the screws used for fixation of femoral neck fracture clinically are mostly compressive hollow screws, and have a variety of structures, for example, hollow screws made of titanium metal or the like which have a shorter triangular thread structure, and most of tail nail heads of the hollow screws made of other materials have circular structure. These screws exist many common clinical problems, such as complex structure, poor biomechanical performance, insufficient mechanical strength and needing secondary removal. Problems, such as internal fixation screw cracking and fracture re-displacement, often occur in the application. For example, CN 205 083 596 U discloses a screw having a screw rod and nut, wherein the screw rod has a screw thread and the nut has a head which is provided with grooves that are symmetrically arranged on the centerline of the screw body. Between the head and the screw thread the screw comprises a smooth bar. US 5 171 279 A discloses a bone screw having distal threaded shank, a smooth shank portion, a rear machine threaded stem, which is threaded for engagement with a nut used to fix a fixation plate. The distal threaded shank is fastened in the vertebra, wherein the rear machine threaded stem is at the outer lining of muscle tissue where the fixation plate 40 is applied. CN 104 352 276 A discloses a bone locking screw that is threaded along its entire length. The screw is fastened to the bone along all of the screw's length.

### SUMMARY OF THE INVENTION

The scope of this invention is defined by the claims. Embodiments in the description relating to methods of treatment are not covered by the claims. The object of the present invention is to provide a porous tantalum metal screw for fixing femoral neck fracture for the problems existing in screws for fixing femoral neck fracture. The porous tantalum metal screw has the advantages of simple structure, better mechanical strength, better biological performance, firm internal fixation performance, higher tissue compatibility and biomechanical properties, and can induce bone tissue formation and facilitate the growth of bone tissues.

The technical solutions of the present invention are as follows:
A porous tantalum metal screw for fracture fixation, comprising a screw body, a front end thread segment is arranged at the front end of the screw body, a rear end thread segment is arranged at the rear end of the screw body, and there is a smooth round bar between the front end thread segment and the rear end thread segment; wherein the thread pitch of the rear end thread segment is smaller than that of the front end thread segment, and the thread depth of the rear end thread segment is greater than that of the front end thread segment, the outer surfaces of two sides of the end of the rear end thread segment are provided with grooves which are symmetrically arranged on the centerline of the screw body, and the angle between the two slopes of the grooves is 30°.

Wherein the porous tantalum metal screw in the technical solution is a solid screw.

Further, the ratio of the thread pitch of the rear end thread segment to that of the front end thread segment is 8-9:10, and the ratio of the thread depth of the rear end thread segment to that of the front end thread segment is 10:8-9.

Further, the ratio of the length of the front end thread segment to that of the screw body is 1:3.5-4.5.

Further, the ratio of the length of the rear end thread segment to that of the screw body is 1: 3.0∼4.5.

Further, the ratio of the length of the groove to that of the screw body is 1: 8∼10.

Further, the threads of both the front end thread segment and the rear end thread segment are wedge-shaped threads. Such design can reduce the self-tapping process before the screw enters, reduce the bone loss and strengthen the internal fixation at the fracture site.

Further, the thread of the front end thread segment is an inch thread with a thread angle of 55°.

Further, the thread of the rear end thread segment is a metric thread with a thread angle of 60°.

Further, of the porous tantalum metal screw of the present invention, the maximum major diameter of thread of the front end thread segment is the same as the major diameter of thread of the rear end thread segment, and the ratio of the major diameter of thread to the diameter of screw round bar is 1.2-1.5:1. The screw round bar is the non-threaded part of the screw.

In the present invention, the screw thread of the rear end thread segment has a smaller thread pitch and a larger thread depth than those of the front end thread segment for the porous tantalum metal screw for fracture fixation, and such design facilitate the holding of the screw to prevent retracting and strengthen the fixation of the fracture site.

Further, a material used for the porous tantalum metal screw is prepared by method of chemical vapor deposition, which reducing tantalum metal compound to tantalum metal powder and evenly depositing the tantalum metal powder on the surface of a graphite carbon skeleton, silicon carbide skeleton or glassy carbon fiber skeleton to form tantalum coating; wherein the tantalum metal compound is one of tantalum chloride and tantalum fluoride; a porosity of the graphite carbon skeleton, silicon carbide skeleton or glassy carbon fiber skeleton is greater than 85%, and the pore size is 200-600µm; a thickness of the tantalum coating is 40-60 µm.

The above material used for preparing the porous tantalum metal screw has a porosity of 85% and a pore diameter of 200-600 µm, and the material is in a reticular structure with at least 80% mutually interlaced polyhedron and has dodecahedron reticular structure with three-dimensional connectivity distribution of pores, which facilitate the growth of bone cells and tissues.

The present invention further provides a method for preparing the porous tantalum metal screw for fracture fixation, comprising the following steps:
(1) etching the graphite carbon skeleton, silicon carbide skeleton or glassy carbon fiber skeleton as stated in claim 9 with dilute hydrochloric acid for 10 to 20 minutes, then washing the graphite carbon skeleton, silicon carbide skeleton or glassy carbon fiber skeleton with water and ethanol in turn and blowing with nitrogen to dry, then placing them into a reaction chamber;
(2) simultaneously introducing a processing gas and hydrogen into the reaction chamber to react for 4-6h at 900-1050°C under a vacuum degree of 10-15Pa;
   wherein: the processing gas comprises tantalum metal compound and carrier gas, the tantalum metal compound is put into a source tank and heated to 120-250°C, and an inert gas at 300°C as the carrier gas is introduced into the reaction chamber;
   the inert gas is one of argon and helium, or a mixture of the two;
   the flow rate of the processing gas is 80-100ml /min; and
   the flow rate of the hydrogen is 100-150 ml/min.

The present invention has the following beneficial effects:
1. The porous tantalum metal screw for fracture fixation of the present invention has a front end thread segment and a rear end thread segment, and the screw thread of the rear end thread segment has a smaller thread pitch and a larger thread depth than those of the front end thread segment, and such design can facilitate the holding of the screw to prevent retracting and strengthen the fixation of the fracture sites.
2. Grooves are symmetrically provided based on the centerline of the screw body on the outer surface at two sides of the end of the rear end thread segment. As a holding part of the tightening tool, both two sides of the holding part have threads to facilitate the adhesion of the screw and the bone cortex, enhancing the fixation of screw on the fixation part and preventing loosening.
3. The screw thread of the front end thread segment of the screw in the present invention is an inch thread with a thread angle of 55°, while the screw thread of the rear end thread segment of the screw is a metric thread with a thread angle of 60°. Such design can strengthen the fixation of screw and prevent screw loosening and retracting.
4. The screw of the present invention can prevent the tail of the screw from being deformed when the screw is threaded in, so that the force reach the front end uniformly, reducing the possibility of deformation and breakage of the screw; the holding part has screw thread, having better grip and fixing effect; the deformation and cracking of the screw thread is avoided when implanting the cortical bone and other hard sclerotin; and the screw does not need to be removed through second operation, so as to lighten the suffering of patients.

The porous tantalum metal screw of the present invention is beneficial to the generating of new bone and replacement of the bone, especially has better clinical effect in the treatment of femoral neck fracture, the material of the porous tantalum metal screw of the present invention has a dodecahedron three-dimensional reticular structure, which is similar to that of trabeculae of cancellous bone, and has high histocompatibility, can induce bone tissue formation, and facilitates bone tissue ingrowth; the material also has strong biomechanical properties, can provide mechanical support, so as to achieve the purposes of inducing and replacing the bone tissues. The porous tantalum metal screw of the present invention has the advantages of simple preparation method, low cost and favorable implementation and clinical application, and is suitable for the needs of the majority of patients.

### BRIEF DESCRIPTION OF THE DRAWINGS

FIG. 1 is a schematic diagram of a porous tantalum metal screw according to the present invention.
FIG. 2 is a schematic diagram of a groove at the end of the rear end thread segment of a porous tantalum metal screw according to the present invention.

Label number: 1- screw body, 11- front end thread segment, 12- rear end thread segment, 13- screw body round bar, 2- groove, L1- length of front end thread segment, L2- length of rear end thread segment. L3- length of screw body round bar.

### DETAILED DESCRIPTIONS OF THE PREFERRED EMBODIMENTS

The following nonrestrictive embodiments can enable those ordinary persons skilled in the art to comprehensively understand the present invention, without limiting the present invention in any way.

### EMBODIMENT 1

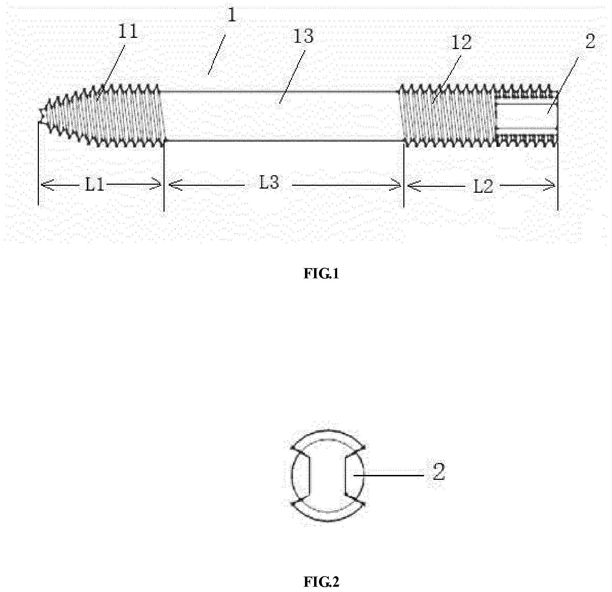

As shown in FIGS. 1-2, a porous tantalum metal screw for fracture fixation comprises a screw body 1, a front end thread segment 11 is arranged at the front end of the screw body, a rear end thread segment 12 is arranged at the rear end of the screw body, a smooth round bar 13 is arranged between the front end thread segment and the rear end thread segment, and the front end thread segment 11, the rear end thread segment 12 and the round bar 13 are integrated structures. Grooves are symmetrically provided based on the centerline of the screw body on the outer surface at two sides of the end of the rear end thread segment, and the angle between two inclined planes of the grooves is 30°, and the screw is a solid screw. The total length of the screw body is 85 mm, the length L1 of the front end thread segment is 20.6 mm, the length L2 of the rear end thread segment is 26 mm, the length L3 of the screw body round bar is 38.4 mm, and the length of the groove is 10mm. The front end thread segment 11 is an inch thread with a thread angle of 55°, a maximum major diameter of the front thread of 10.4 mm, a maximum minor diameter of 7 mm, and a pitch of 4 mm. The rear end thread segment 12 is a metric thread with a thread angle of 60°, the major diameter of the thread is 10.4mm, the maximum minor diameter is 8 mm, and the pitch is 3.5mm;

In particular applications, the specific size of the screw can be appropriately adjusted according to the size and tissues of the implantation site, to achieve the best therapeutic effect.

### EMBODIMENT 2

The porous tantalum metal screw as stated in the Embodiment 1 can be made by the following method:
(1) the graphite carbon skeleton, silicon carbide skeleton, or glassy carbon fiber skeleton with a porosity of 85% is etched with dilute hydrochloric acid for 10 minutes, then successively washed with water and ethanol, dried with nitrogen gas, and placed into a reaction chamber;
(2) The tantalum chloride powder with a particle size of 400 mesh is put into a source tank and heated to 150°C, and a high-temperature argon gas (300°C) is introduced into the reaction chamber as a carrier gas. the flow rate of the carrier gas is 100 ml/ min, the reaction chamber temperature is 1050°C, and the reaction chamber vacuum degree is 10Pa; Hydrogen is introduced at a flow rate of 120ml/min while the tantalum chloride is introduced into the reaction chamber using argon gas as the carrier gas, and the reduction reaction is carried out for 4 hours, so that the tantalum metal reduced to metal powder is uniformly deposited on the surface of the skeleton of the graphite carbon, silicon carbide or glassy carbon fiber and their pores' surface to obtain the finished product of porous tantalum metal screw of the present invention.

The material of porous tantalum metal screw body has a porosity of 85%, an elastic modulus of 10 GPa and a maximum compressive strength of 35 MPa, wherein the porosity is detected according to the national standard GB/T 21650.1-2008, and the elastic modulus is detected according to the national standard GB/T 22315-2008.

The material of porous tantalum metal screw body has a dodecahedral reticular structure with three-dimensionally interconnected distribution of pores, which is similar to that of cancellous bone trabeculae, and has high histocompatibility, can induce bone tissue formation and facilitate bone tissues ingrowth. In addition, the material has strong biomechanical properties and can provide mechanical support, so as to achieve the purpose of promoting fracture healing and repair and filling bone defects.

In the foregoing preparation method, the graphite carbon skeleton, silicon carbide skeleton or glass carbon skeleton is first processed into a shape described in FIG.1, and then the porous tantalum metal screw of the present invention is prepared through the steps (1) to (2).

## Claims

1. A porous tantalum metal screw for fracture fixation, comprising a screw body (1); a front end thread segment (11) is arranged at the front end of the screw body (1), a rear end thread segment (12) is arranged at the rear end of the screw body (1), and there is a smooth round bar (13) between the front end thread segment (11) and the rear end thread segment (12); wherein a thread pitch of the rear end thread segment (12) is smaller than that of the front end thread segment (11), and a thread depth of the rear end thread segment (12) is greater than that of the front end thread segment (11), outer surfaces of two sides of the end of the rear end thread segment (12) are provided with grooves (2) which are symmetrically arranged on a centerline of the screw body (1), and an angle between the two inclined planes of the groove (2) is 30°.

2. The porous tantalum metal screw according to claim 1, wherein the ratio of the thread pitch of the rear end thread segment (12) to that of the front end thread segment (11) is 8-9:10, and the ratio of the thread depth of the rear end thread segment (12) to that of the front end thread segment (11) is 10:8-9.

3. The porous tantalum metal screw according to claim 1, wherein the ratio of the length of the front end thread segment (11) to that of the screw body (1) is 1:3.5-4.5.

4. The porous tantalum metal screw according to claim 1, wherein the ratio of the length of the rear end thread segment (12) to that of the screw body (1) is 1: 3.0∼4.5.

5. The porous tantalum metal screw according to claim 1, wherein the ratio of the length of the groove (2) to that of the screw body (1) is 1: 8∼10.

6. The porous tantalum metal screw according to claim 1, wherein the threads of both the front end thread segment (11) and the rear end thread segment (12) are wedge-shaped threads.

7. The porous tantalum metal screw according to claim 1, wherein the thread of the front end thread segment (11) is an inch thread with a thread angle of 55°.

8. The porous tantalum metal screw according to claim 1, wherein the thread of the rear end thread segment (12) is a metric thread with a thread angle of 60°.

9. The porous tantalum metal screw according to claim 1, wherein a material used for the screw is prepared by method of chemical vapor deposition, which reducing tantalum metal compound to tantalum metal powder, and uniformly depositing the tantalum metal powder on the surface of a graphite carbon skeleton, silicon carbide skeleton or glassy carbon fiber skeleton to form tantalum coating; wherein the tantalum metal compound is one of tantalum chloride and tantalum fluoride, a porosity of the graphite carbon skeleton, silicon carbide skeleton or glassy carbon fiber skeleton is greater than 85%, and the pore size is 200-600 µm; a thickness of the tantalum coating is 40-60 µm.

10. A method for preparing the porous tantalum metal screw according to claim 9, comprising the following steps:
(1) etching the graphite carbon skeleton, silicon carbide skeleton or glassy carbon fiber skeleton according to claim 9 with dilute hydrochloric acid for 10-20min, then washing the graphite carbon skeleton, silicon carbide skeleton or glassy carbon fiber skeleton with water and ethanol in turn and blowing with nitrogen to dry, then placing the graphite carbon skeleton, silicon carbide skeleton or glassy carbon fiber skeleton into a reaction chamber;
(2) simultaneously introducing a processing gas and hydrogen into the reaction chamber to react for 4-6h at 900-1050°C under a vacuum degree of 10-15Pa;
wherein: the processing gas comprises tantalum metal compound and carrier gas, the tantalum metal compound is put into a source tank and heated to 120-250°C, and an inert gas at 300°C as the carrier gas is introduced into the reaction chamber;
wherein the inert gas is one of argon and helium, or a mixture of the two;
a flow rate of the processing gas is 80-100ml /min; and
a flow rate of the hydrogen is 100-150 ml/min.

## Patentansprüche

1. Poröse Tantalmetallschraube zur Frakturfixierung, umfassend einen Schraubenkörper (1); ein vorderes Gewindesegment (11), das am vorderen Ende des Schraubenkörpers (1) angeordnet ist, ein hinteres Gewindesegment (12), das am hinteren Ende des Schraubenkörpers (1) angeordnet ist, und einen glatten Rundstab (13) zwischen dem vorderen Gewindesegment (11) und dem hinteren Gewindesegment (12); wobei eine Gewindesteigung des Gewindesegments (12) am hinteren Ende kleiner ist als die des Gewindesegments (11) am vorderen Ende und eine Gewindetiefe des Gewindesegments (12) am hinteren Ende größer ist als die des Gewindesegments (11) am vorderen Ende, Außenflächen von zwei Seiten des Endes des Gewindesegments (12) am hinteren Ende mit Nuten (2) versehen sind, die symmetrisch auf einer Mittellinie des Schraubenkörpers (1) angeordnet sind, und ein Winkel zwischen den beiden geneigten Ebenen der Nut (2) 30° beträgt.

2. Poröse Tantalmetallschraube nach Anspruch 1, wobei das Verhältnis der Gewindesteigung des hinteren Gewindesegments (12) zu der des vorderen Gewindesegments (11) 8-9:10 beträgt und das Verhältnis der Gewindetiefe des hinteren Gewindesegments (12) zu der des vorderen Gewindesegments (11) 10:8-9 beträgt.

3. Poröse Tantalmetallschraube nach Anspruch 1, wobei das Verhältnis der Länge des vorderen Gewindesegments (11) zu der des Schraubenkörpers (1) 1:3,5-4,5 beträgt.

4. Poröse Tantalmetallschraube nach Anspruch 1, wobei das Verhältnis der Länge des hinteren Gewindesegments (12) zu der des Schraubenkörpers (1) 1:3,0∼4,5 beträgt.

5. Poröse Tantalmetallschraube nach Anspruch 1, wobei das Verhältnis der Länge der Nut (2) zu der des Schraubenkörpers (1) 1:8∼10 beträgt.

6. Poröse Tantalmetallschraube nach Anspruch 1, wobei die Gewinde sowohl des vorderen Gewindesegments (11) als auch des hinteren Gewindesegments (12) keilförmige Gewinde sind.

7. Poröse Tantalmetallschraube nach Anspruch 1, wobei das Gewinde des vorderen Gewindesegments (11) ein Zollgewinde mit einem Gewindewinkel von 55° ist.

8. Poröse Tantalmetallschraube nach Anspruch 1, wobei das Gewinde des hinteren Gewindesegments (12) ein metrisches Gewinde mit einem Gewindewinkel von 60° ist.

9. Poröse Tantalmetallschraube nach Anspruch 1, wobei ein für die Schraube verwendetes Material durch ein Verfahren der chemischen Gasphasenabscheidung hergestellt wird, bei dem eine Tantalmetallverbindung zu Tantalmetallpulver reduziert wird und das Tantalmetallpulver gleichmäßig auf der Oberfläche eines Graphit-Kohlenstoff-Skeletts, Siliziumkarbid-Skeletts oder glasartigen Kohlenstofffaser-Skeletts abgeschieden wird, um eine Tantal-Beschichtung zu bilden; wobei die Tantalmetallverbindung eines von Tantalchlorid und Tantalfluorid ist, die Porosität des Graphit-Kohlenstoff-Skeletts, Siliziumkarbid-Skeletts oder glasartigen Kohlenstofffaser-Skeletts größer als 85% ist und die Porengröße 200-600 µm beträgt; eine Dicke der Tantal-Beschichtung 40-60 µm beträgt.

10. Verfahren zur Herstellung der porösen Tantalmetallschraube nach Anspruch 9, umfassend die folgenden Schritte:
(1) Ätzen des Graphitkohlenstoffskeletts, Siliciumcarbidskeletts oder Glaskohlenstofffaserskeletts nach Anspruch 9 mit verdünnter Salzsäure für 10-20 min, dann Waschen des Graphitkohlenstoffskeletts, Siliciumcarbidskeletts oder Glaskohlenstofffaserskeletts abwechselnd mit Wasser und Ethanol und Blasen mit Stickstoff zum Trocknen, dann Einbringen des Graphitkohlenstoffskeletts, Siliciumcarbidskeletts oder Glaskohlenstofffaserskeletts in eine Reaktionskammer;
(2) gleichzeitiges Einführen eines Prozessgases und von Wasserstoff in die Reaktionskammer, um für 4-6h bei 900-1050°C unter einem Vakuumgrad von 10-15Pa zu reagieren;
wobei das Prozessgas eine Tantalmetallverbindung und ein Trägergas umfasst, die Tantalmetallverbindung in einen Quellentank gegeben und auf 120-250°C erhitzt wird, und ein Inertgas bei 300°C als das Trägergas in die Reaktionskammer eingeführt wird;
wobei das Inertgas eines von Argon und Helium oder eine Mischung der beiden ist; eine Strömungsrate des Prozessgases 80-100 ml/min beträgt; und
eine Strömungsrate des Wasserstoffs 100-150 ml/min beträgt.

## Revendications

1. Vis métallique poreuse en tantale pour la fixation de fractures, comprenant un corps de vis (1) ; un segment de filetage d'extrémité avant (11) est disposé à l'extrémité avant du corps de vis (1), un segment de filetage d'extrémité arrière (12) est disposé à l'extrémité arrière du corps de vis (1), et il y a une barre ronde lisse (13) entre le segment de filetage d'extrémité avant (11) et le segment de filetage d'extrémité arrière (12) ; dans lequel un pas de filetage du segment de filetage d'extrémité arrière (12) est inférieur à celui du segment de filetage d'extrémité avant (11), et une profondeur de filetage du segment de filetage d'extrémité arrière (12) est supérieure à celle du segment de filetage d'extrémité avant (11), les surfaces extérieures de deux côtés de l'extrémité du segment de filetage d'extrémité arrière (12) sont pourvues de rainures (2) qui sont disposées symétriquement sur une ligne centrale du corps de vis (1), et un angle entre les deux plans inclinés de la rainure (2) est de 30°.

2. Vis en métal de tantale poreux selon la revendication 1, dans laquelle le rapport du pas de filetage du segment de filetage d'extrémité arrière (12) sur celui du segment de filetage d'extrémité avant (11) est de 8-9:10, et le rapport de la profondeur de filetage du segment de filetage d'extrémité arrière (12) sur celle du segment de filetage d'extrémité avant (11) est de 10:8-9.

3. Vis en métal de tantale poreux selon la revendication 1, dans laquelle le rapport de la longueur du segment de filetage d'extrémité avant (11) sur celle du corps de vis (1) est de 1:3,5-4,5.

4. Vis en métal de tantale poreux selon la revendication 1, dans laquelle le rapport de la longueur du segment de filetage d'extrémité arrière (12) sur celle du corps de vis (1) est de 1 : 3,0∼4,5.

5. Vis en métal de tantale poreux selon la revendication 1, dans laquelle le rapport de la longueur de la rainure (2) sur celle du corps de la vis (1) est de 1 : 8∼10.

6. Vis en métal de tantale poreux selon la revendication 1, dans laquelle les filets du segment de filetage d'extrémité avant (11) et du segment de filetage d'extrémité arrière (12) sont des filets en forme de coin.

7. Vis en métal de tantale poreux selon la revendication 1, dans laquelle le filetage du segment de filetage d'extrémité avant (11) est un filetage en pouces avec un angle de filetage de 55°.

8. Vis en métal de tantale poreux selon la revendication 1, dans laquelle le filetage du segment fileté d'extrémité arrière (12) est un filetage métrique avec un angle de filetage de 60°.

9. Vis en métal de tantale poreux selon la revendication 1, dans laquelle un matériau utilisé pour la vis est préparé par un procédé de dépôt chimique en phase vapeur, qui réduit le composé de métal de tantale en poudre de métal de tantale, et dépose uniformément la poudre de métal de tantale sur la surface d'un squelette de carbone graphite, d'un squelette de carbure de silicium ou d'un squelette de fibre de carbone vitreux pour former un revêtement de tantale ; dans lequel le composé métallique de tantale est l'un des chlorure de tantale et fluorure de tantale, une porosité du squelette de carbone graphite, du squelette de carbure de silicium ou du squelette de fibre de carbone vitreux est supérieure à 85 %, et la taille des pores est de 200-600 µm ; une épaisseur du revêtement de tantale est de 40-60 µm.

10. Procédé de préparation de la vis métallique poreuse en tantale selon la revendication 9, comprenant les étapes suivantes :
(1) gravure du squelette en carbone graphite, du squelette en carbure de silicium ou du squelette en fibre de carbone vitreuse selon la revendication 9 avec de l'acide chlorhydrique dilué pendant 10-20min, puis lavage du squelette en carbone graphite, du squelette en carbure de silicium ou du squelette en fibre de carbone vitreuse avec de l'eau et de l'éthanol tour à tour et soufflage avec de l'azote pour sécher, puis placement du squelette en carbone graphite, du squelette en carbure de silicium ou du squelette en fibre de carbone vitreuse dans une chambre de réaction ;
(2) introduire simultanément un gaz de traitement et de l'hydrogène dans la chambre de réaction pour réagir pendant 4-6h à 900-1050°C sous un degré de vide de 10-15Pa ;
dans lequel : le gaz de traitement comprend un composé métallique de tantale et un gaz porteur, le composé métallique de tantale est placé dans un réservoir source et chauffé à 120-250°C, et un gaz inerte à 300°C comme gaz porteur est introduit dans la chambre de réaction ;
dans lequel le gaz inerte est l'un parmi l'argon et l'hélium, ou un mélange des deux ;
un débit du gaz de traitement est de 80-100ml /min ; et
un débit de l'hydrogène est de 100-150 ml/min.
